(19) Europäisches Patentamt
European Patent Office
Office européen des brevets

(11) Publication number: **0 274 795 B1**

(12) **EUROPEAN PATENT SPECIFICATION**

(45) Date of publication of patent specification: **21.08.91**

(51) Int. Cl.5: **C07C 67/38**, C07C 69/24

(21) Application number: **87202537.4**

(22) Date of filing: **15.12.87**

(54) **Process for the carbonylation of olefinically unsaturated compounds with a palladium catalyst.**

(30) Priority: **24.12.86 NL 8603302**

(43) Date of publication of application:
**20.07.88 Bulletin 88/29**

(45) Publication of the grant of the patent:
**21.08.91 Bulletin 91/34**

(84) Designated Contracting States:
**BE DE ES FR GB IT NL**

(56) References cited:
**EP-A- 0 063 818**
**EP-A- 0 106 379**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Drent, Eit**
**Badhuisweg 3**
**NL-1031 CM Amsterdam(NL)**

(74) Representative: **Aalbers, Onno et al**
**P.O. Box 302**
**NL-2501 CH The Hague(NL)**

EP 0 274 795 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a process for the carbonylation of olefinically unsaturated compounds with a palladium catalyst. In particular, the present invention relates to an improved process for the conversion of olefinically unsaturated compounds with carbon monoxide in the presence of a hydroxyl-group-containing compound such as water, an alcohol or a carboxylic acid under formation of carboxylic acids, esters or carboxylic acid anhydrides respectively.

The hitherto known processes have the drawback that relatively high pressures are necessary, while in addition the conversion rates are relatively low, which leads to long reaction times. This makes them unattractive for application on a technical scale. Such a process is (for example) known from US 3,437,676.

In European patent specification No. 106,379 a process is described for the carbonylation of propene in the presence of water or an alkanol and a catalyst, composed of palladium or a compound thereof, a triaryl phosphine and an acid as promotor, wherein although the reaction rate appears to be considerably increased relative to the hitherto known processes, part of the promoter acid is lost during the course of the reaction, which causes deactivation of the catalyst system used.

Surprisingly, it has now been found as the result of extensive research and development that the reaction rate of the said conversion can be maintained for a considerable time at the level desired by using a catalyst stabilizer, to be described hereinafter in more detail, in appropriate quantities.

The present invention therefore relates to a process for the carbonylation of olefinically unsaturated compounds with carbon monoxide in the presence of a hydroxyl-group-containing compound such as water, an alcohol or a carboxylic acid, a palladium catalyst, an organic phosphine according to the formula

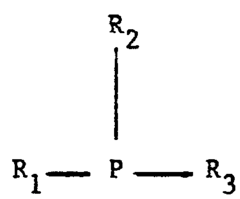

I

wherein $R_1$, $R_2$ and $R_3$ each represent an optionally substituted aryl group, and an acid with a pKa value ≦ 2, with the exception of hydrohalogenic acids and carboxylic acids, as promoter, characterized in that a catalyst stabilizer is used.

Catalyst stabilizers which can be used according to one of the embodiments of the process according to the present invention are, for example, compounds according to the general formula

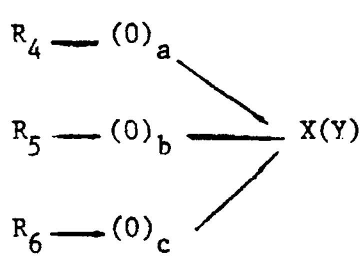

II

wherein X represents an element of group Va with a valency greater than 3, selected from P, As or Sb, wherein Y represents an element of group VIa, selected from O, S or Se, wherein either a, b and c are 0 or 1, and wherein $R_4$, $R_5$ and $R_6$ are the same or different and represent optionally substituted hydrocarbyl groups, and wherein either a and b = 0 and c = 0 or 1 and $R_4$ and $R_5$ together with X form a heterocyclic group, or a, b and c = 0 and $R_4$, $R_5$ and $R_6$ together with X form an optionally substituted heterocyclic group.

According to another embodiment of the process according to the present invention, catalyst stabilizers can be used according to the general formula

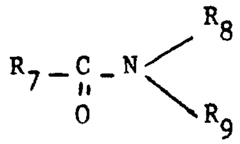

III

wherein $R_7$ represents an optionally substituted hydrocarbyl group and wherein $R_8$ and $R_9$ are each the same or different and have the aforesaid meaning of $R_7$ or together with N form an optionally substituted heterocyclic ring, or wherein $R_7$ and $R_8$ or $R_9$ together with the moiety

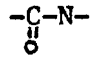

form a heterocyclic ring such as a pyrrolidon or caprolactam ring.

The aforesaid optionally substituted hydrocarbyl groups in the definitions of $R_4$ to $R_9$ can be alkyl, cycloalkyl, aryl, aralkyl or alkaryl groups containing up to 30 carbon atoms. If $R_4$ and $R_5$ or $R_5$ and $R_6$ or $R_8$ and $R_9$ or $R_7$ and $R_8$ or $R_9$ form a heterocyclic group either with X in formula II or with N or -C-N- in formula III, the hydrocarbyl residue of the heterocyclic group can contain up to 21 carbon atoms. The preferred compounds according to formula II are those wherein a, b and c = 0, wherein X = P, wherein Y = 0 or S and wherein $R_4$, $R_5$ and $R_6$ represent alkyl groups which comprise 1-4 carbon atoms, or cycloalkyl, aryl or aralkyl groups which comprise 5-12 carbon atoms and are preferably alkyl, phenyl, cyclohexyl or benzyl groups. In more preferred groups according to formula II, Y represents oxygen, while $R_4$, $R_5$ and $R_6$ represent alkyl groups or Y represents sulphur, while $R_4$, $R_5$ and $R_6$ represent optionally substituted phenyl groups. The substituents, if any, on the hydrocarbyl groups and/or the heterocyclic groups should in fact be inert in the reaction medium. Suitable substituents are, for example, chlorine, alkoxy groups, carboxylic acid (ester) groups or sulphonic or sulphoxide groups.

Examples of suitable stabilizers according to formula II are oxides, sulphides or selenides of tertiary phosphines or the corresponding arsenic or antimony derivatives such as trimethyl phosphine oxide, triethyl phosphine oxide, tri-n-propyl phosphine oxide, tri-n-butyl phosphine oxide, triphenyl phosphine oxide, tri-p-tolyl phosphine oxide, tri-p-tolyl phosphine sulphide, tricyclohexyl phosphine oxide, diphenyl ethyl phosphine oxide, tri(1-naphthyl) phosphine oxide, tri(1-naphthyl) phosphine sulphide, 1-phenyl phosphalane sulphide, 1-phenyl phosphorinane sulphide, trimethyl phosphine sulphide, tri(4-chlorophenyl) phosphine sulphide, triphenyl phosphine sulphide, tricyclohexyl phosphine sulphide, tri-n-butyl phosphine sulphide, triphenyl phosphine selenide, tri(1-naphthyl) phosphine selenide and triphenyl arsine sulphide. Of these, tri-p-tolyl phosphine sulphide, triphenyl phosphine sulphide, tricyclohexyl phosphine sulphide, tri-n-butyl phosphine oxide, trimethyl phosphine oxide, tri-n-propyl phosphine oxide and triethyl phosphine oxide are preferred.

Other examples of suitable stabilizers according to formula II are alkyl and aryl esters of phosphoric acid, phosphonic acid and phosphinic acids and their arsenic or antimony analogues such as trimethyl phosphate, triethyl phosphate, tri-n-butyl phosphate, triphenyl phosphate, dimethyl methyl phosphonate, 1,5-dimethyl bicyclo(3,2,1,) octyl phosphonate, diethyl methyl phosphonate and methyl diphenyl phosphonate. Preferably, phosphonates are used.

It will be appreciated that according to another embodiment of the process according to the present invention, stabilizers can also be used which comprise two or more moieties represented by formula II. Examples of such stabilizers are

tetraphenyl ethylene diphosphine disulphide,
tetraphenyl methylene diphosphine disulphide
tetramethyl ethylene diphosphine dioxide,
tetraphenyl propylene diphosphine disulphide,
tetraphenyl butylene diphosphine disulphide,
tetraethyl ethylene diphosphine dioxide,
tetrapropyl ethylene diphosphine dioxide,
tetraethyl propylene diphosphine dioxide,
tetraethyl butylene diphosphine dioxide,
tetramethyl butylene diphosphine dioxide and
tetramethyl propylene diphosphine dioxide.

Preferred stabilizers according to general formula III are those wherein $R_7$, $R_8$ and $R_9$ represent an alkyl

group of 1-4 carbon atoms, a cycloalkyl, cycloalkaryl, aryl or aralkyl group comprising 5-12 carbon atoms, and preferably alkyl, phenyl, benzyl or cyclohexyl groups, or wherein $R_7$, $R_8$ or $R_9$ together with the moiety

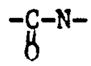

form a heterocyclic ring.

Examples of suitable stabilizers according to formula III are
N,N'-dimethyl acetamide, N,N'-diethyl acetamide,
N,N'-diethyl propionamide, N,N'-diethyl butamide,
N,N'-dipropyl propionamide, N,N'-di(isopropyl)-propionamide, N,N'-di(isopropyl) acetamide,
N,N'-diphenyl acetamide, N-methyl pyrrolidone and
N-methyl caprolactam.

It will be appreciated that according to another embodiment of the process according to the present invention, stabilizers can also be used which comprise two or more moieties represented by formula III. Examples of such stabilizers are
N,N,N'N'-tetramethyl malonamide,
N,N,N'N'-tetraethyl malonamide,
N,N,N'N'-tetramethyl succinamide and
N,N,N'N'-tetraethyl succinamide,

It will be appreciated that each of the aforesaid stabilizers can be used as such or in combination with one or more others.

The quantity of catalyst stabilizer according to formulae II and III that can be used in the process according to the present invention can vary from 0.01 mol to 200 mol per gram-atom palladium.

The acids that can be used as promoters in the process according to the present invention preferably comprise a non-coordinating anion, by which is meant that little or no covalent interaction takes place between palladium and the appropriate anion. Typical examples of such anions are $PF_6^-$, $SbF_6^-$, $BF_4^-$ and $ClO_4^-$. Preferably used acids are, for example, sulphonic acids and acids that can be formed, possibly in situ, by the interaction of a Lewis acid such as $BF_3$, $AsF_5$, $SbF_5$, $PF_5$, $TaF_5$ or $NbF_5$ with a Brönsted acid such as a hydrohalogenic acid, in particular hydrofluoric acid, fluorosulphonic acid, phosphoric acid or sulphuric acid. Specific examples of the latter type of acids are $H_2SiF_6$, $HBF_4$, $HPF_6$ and $HSbF_6$. Typical sulphonic acids that can be used are fluorosulphonic acid, chlorosulphonic acid and the sulphonic acids specified below. A preferred group of acids has the general formula

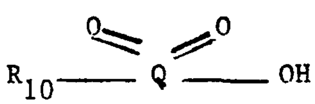 IV

wherein Q represents sulphur or chlorine and where, if Q is chlorine, $R_{10}$ represents oxygen and if Q is sulphur, $R_{10}$ represents an OH-group or an optionally substituted hydrocarbyl group.

If the aforesaid acids are used in the process according to the present invention, the anions of the acids are considered as non-coordinating.

The carbonylation of the olefinically unsaturated compounds should preferably be carried out in the presence of both an acid as specified hereinabove and at least 5 mol of an appropriate phosphine. In the acids according to formula IV, the optionally substituted hydrocarbyl group in the definition of $R_{10}$ is preferably an alkyl, aryl or aralkyl group with 1-30 and preferably 1-14 carbon atoms. The hydrocarbyl group may be substituted with, for example, halogen atoms, in particular fluorine atoms. Examples of suitable acids according to formula IV are perchloric acid, sulphuric acid, 2-hydroxypropane-2-sulphonic acid, p-toluene sulphonic acid and tritfluoromethane sulphonic acid, of which the last two acids are preferred. The acid according to formula IV can also be an ion exchanger comprising sulphonic acid groups such as Amberlite 252 H [R]. In that case, the hydrocarbyl group $R_{10}$ consists of a polymeric hydrocarbyl group substituted with sulphonic acid groups, for example a polystyrene polymer with sulphonic acid groups.

[R] the word Amberlite 252 H is a Registered Trade Mark

The quantity of acid present in the reaction mixture is preferably 0.01-150, and in particular 0.1-100, and more preferably 1-50 equivalents per gram-atom palladium The acid can optionally be formed in situ, for example by hydrolysis of an ester such as an alkyl ester of a sulphonic acid or by the reaction of a ketone with $SO_2$ and water.

It will be appreciated that by applying the process according to the present invention, high reaction rates can be achieved which, during the course of the carbonylation reaction, can be maintained practically at the initial level, thus leading to shorter overall reaction times and making this process ideally suited for continuous operation. Moreover, the stabilizing effect leads to reduced catalyst consumption and thus to lower costs for the preparation of, for example, methyl propionate from ethene, which makes this process a considerable improvement for batchwise, semi-continuous and continuous operation.

The olefinically unsaturated compounds to be converted can be unsubstituted or substituted alkenes or cycloalkenes, which preferably contain 2-30 and in particular 2-20 carbon atoms. Preferably, these starting compounds contain 1-3 double bonds. The alkenes or cycloalkenes can, for example, be substituted with one or more halogen atoms or cyano, ester, alkoxyl, hydroxyl, carboxyl or aryl groups. If the substituents are not inert under the reaction conditions, the carbonylation reaction may be accompanied by other reactions. For example, the carbonylation of an alkyl alcohol may be accompanied by esterification of the hydroxyl group. Examples of suitable olefinic compounds are ethene, propene, butene-1, butene-2, isobutene, the isomeric pentenes, hexenes, octenes and dodecenes, 1,5-cyclooctadiene, cyclododecene, 1,5,9-cyclododecatriene, allyl alcohol, methyl acrylate, ethyl acrylate, methyl methacrylate, acrylonitrile, acrylamide, N,N-dimethyl acrylamide, vinyl chloride, allyl chloride, acroleine, oleic acid, linoleic acid, methyl allyl ether and styrene.

The alcohols or carboxylic acids used in the process according to the present invention can be aliphatic, cycloaliphatic or aromatic and can be substituted with one or more substituents such as mentioned hereinbefore in connection with the olefinically unsaturated compounds which can be used as starting material. The alcohol can therefore also be a phenol. The alcohols or carboxylic acids preferably contain no more than 20 carbon atoms. Examples of suitable alcohols or carboxylic acids are methanol, ethanol, propanol, isobutanol, tert. butanol, stearyl alcohol, benzyl alcohol, cyclohexanol, allyl alcohol, chlorocapryl alcohol, ethylene glycol, 1,2-propane diol, 1,4-butane diol, glycerol, polyethylene glycol, 1,6-hexane diol, phenol, cresol, acetic acid, propionic acid, butyric acid, capronic acid, trimethyl acetic acid, benzoic acid, caprylic acid, adipic acid and hydroxycapronic acid.

Particular preference is given to alkanols and carboxylic acids having 1-10 carbon atoms. If the alcohol or the carboxylic acid comprises more than one hydroxyl group or carboxylic acid group, various products can be formed, depending on the molar ratios of the reagents. For example, a monoester or a diester can be formed from glycerol, depending on the quantity of olefinically unsaturated compound used.

The products formed by the process according to the present invention can, if desired, be converted further. For example, if the carbonylation of an olefin is carried out in the presence of water, a carboxylic acid can be formed that can, by the reaction of an additional quantity of olefin, form a carboxylic acid anhydride. If the carbonylation is carried out in the presence of an alcohol, an ester is formed which, if water is also present, can hydrolyze to form an acid and an alcohol, which can then again each react with an olefin. If the carbonylation is carried out in the presence of a carboxylic acid, an acid anhydride is formed which, if water is also present, can hydrolyze to form one or more carboxylic acids which in turn can react further with a quantity of olefin. The reaction of an alkane carboxylic acid comprising $n+1$ carbon atoms with an olefin comprising n carbon atoms forms the symmetric anhydride of the alkane carboxylic acid with $n+1$ carbon atoms. This anhydride can be optionally hydrolyzed, whereby half of the carboxylic acid formed as a product can be collected and the other half can be returned to the carbonylation reactor. The process thus leads to the conversion of an olefin with n carbon atoms to a carboxylic acid of $n+1$ carbon atoms.

Both homogeneous and heterogeneous palladium catalysts can be used in the process according to the present invention. Homogeneous catalysts are, however, preferred. Suitable homogeneous catalysts are palladium salts of nitric acid, sulphuric acid or alkane carboxylic acids with not more than 12 carbon atoms per molecule. Salts of hydrohalogenic acids can in principle also be be used, but these have the drawback that the halide ion can have a corrosive effect. Preferably, palladium acetate is used as catalyst. Palladium complexes, such as palladium acetyl acetonate, tetrakis triphenyl phosphine palladium, bis-tri-0-tolyl phosphine palladium acetate or bis-triphenyl phosphine palladium sulphate, can also be used. Palladium on active carbon or bonded to an ion-exchanger, for example an ion exchanger comprising sulphonic acid groups, are examples of suitable heterogeneous catalysts.

The quantity of the palladium compound is not critical. Preferably, quantities are used in the range of $10^{-5}$ to $10^{-1}$ gram-atom palladium per mol olefinically unsaturated compound.

The substituted or unsubstituted aryl groups $R_1$, $R_2$ and $R_3$ according to formula I preferably comprise not more than 18 carbon atoms, and in particular 6-14 carbon atoms. Examples of suitable $R_1$, $R_2$ and $R_3$ groups are the naphthyl group and more preferably the phenyl group. Suitable substituents are halogen atoms and alkyl, aryl, alkoxy, carboxy, carbalkoxy, acyl, trihalogenomethyl, cyano, dialkyl amino, sulphonyl alkyl and alkanoyloxy groups.

Examples of suitable phosphines are triphenyl phosphine, tri-p-methoxyphenyl phosphine, o-diphenyl phosphino benzoic acid and in particular triphenyl phosphine. The phosphine is used in a quantity of at least 5 mol and preferably 10-150 mol per gram-atom palladium. If the palladium catalyst to be applied already comprises phosphine, this should be taken into account when calculating the total amount of phosphine to be used.

In the process according to the present invention the carbon monoxide can be used in a pure form or diluted with an inert gas, such as hydrogen, nitrogen, noble gases or carbon dioxide. In general, the presence of more than 10% hydrogen is undesirable, since it can cause hydrogenation of the olefinic compound under the reaction conditions. The carbonylation according to the present invention is preferably carried out at a temperature in the range of 50 to 200 °C and in particular between 75 and 150 °C. The total pressure preferably lies between 1 and 100, and more in particular between 20 and 75 bar overpressure.

The molar ratio of the olefinically unsaturated compound to water, alcohol or carboxylic acid is not critical. The molar ratio of the hydroxyl-group-containing compound to olefinic double bonds can lie, for example, between 0.1:1 and 10:1. If a mono-olefin and/or water, a monohydric alcohol or a monobasic acid is applied, the use of an excess of the aforementioned hydroxylcompound is preferred. If, however, a polyhydric alcohol or a polybasic acid is used to prepare a polyester or a polyanhydride, it will in general be necessary to use an excess of the olefinic compound.

The process according to the present invention can be carried out batchwise, continuously or semi-continuously.

In general, no separate solvent is required, since there will usually be an excess of one of the reactants, for example the alcohol, which can also function as solvent. If desired, however, a solvent can be used, for example benzene, the xylenes, toluene, anisole, diisopropyl sulphone, sulfolan, acetone, chloroform, methyl isobutyl ketone, diglym (diethylene glycol dimethyl ether), diphenyl ether or diisopropyl ether. The primary reaction product of the carbonylation reaction can also be used as solvent.

It will be appreciated that the present invention also relates to the aforesaid catalyst systems, which are used for the selective conversion of olefinically unsaturated compounds, either as such or in the form of a solution in one or more appropriate solvents.

Although the application of some of the compounds according to formula II in catalyst systems is known from, for example, European patent application No. 108,437, it will be appreciated that the catalyst systems used herein comprise completely different components (bromide or iodide sources), while the reaction system comprises entirely different reactants and the conversion is of a quite different type.

Therefore, the present invention also relates to catalyst systems for the selective carbonylation of olefinically unsaturated compounds in the presence of a hydroxyl-group-containing compound comprising:

a) a palladium catalyst,

b) an organic phosphine,

c) an acid with a pKa value ≦ 2, with the exception of hydrohalogenic acids and carboxylic acids, as promoter, and

d) a catalyst stabilizer.

The catalyst stabilizers which can be used in the catalyst systems according to the present invention comprise compounds according to the general formulae II and III, wherein $R_4$ to $R_9$, X and Y represent any of the meanings as defined hereinbefore.

It will be appreciated that according to another embodiment of the present invention the catalyst systems can comprise stabilizers which comprise two or more groups represented by formulae II and III.

The quantity of catalyst stabilizer according to formulae II and III in the catalyst systems according to the present invention can vary from 0.01 mol to 200 mol per gram atom palladium. The acids that can be used as promotors in the catalyst systems preferably comprise a non-coordination anion and are those mentioned hereinbefore.

A preferred group of acids are those according to the general formula IV as described hereinbefore.

The invention will now be illustrated by the following Examples.

Example 1

[R] the word Hastelloy is a Registered Trade Mark

A 250 ml magnetically stirred Hastelloy C [R]autoclave was filled with 10 ml methanol, 50 ml methyl propionate, 0.1 mmol palladium acetate, 3 mmol triphenyl phosphine, 2 mmol p-toluene sulphonic acid and 10 mmol tributyl phosphine oxide. The autoclave was purged with carbon monoxide and filled with ethene to a pressure of 20 bar and carbon monoxide to a pressure of 30 bar, closed and heated to a temperature of 110 °C. After a reaction time of 5 hours, the autoclave contents were analyzed by gas-liquid chromatography. The starting acid (p-toluene suphonic acid) still present was determined with the aid of potentiometric titration with a base (NaOH/methanol mixtures). The methanol to methyl propionate conversion was 48%. The selectivity of the ethene to methyl propionate conversion was found to be almost 100%, while the average conversion rate was 240 mol/g.at.Pd hour.

Example 2

In a virtually analogous manner to that described in Example 1, an experiment was carried out in which 5 mmol instead of 10 mmol tributyl phosphine oxide was added. The reaction time was 2.5 hours. The methanol to methyl propionate conversion was 48%. The initially added acid was found (by means of the method mentioned in Example 1) to be still present In its entirety in the reaction mixture. The selectivity of the ethene to methyl propionate conversion was found to be almost 100%, while the average conversion rate was 480 mol/g.at.Pd hour.

Example 3

In a virtually analogous manner as described in Example 1, an experiment was carried out in which 2 mmol instead of 10 mmol tributyl phosphine oxide was added. The reaction time was 2.5 hours. The initially added acid was found (by the aforesaid method) to be still present almost in its entirety ( 95%) in the reaction mixture. The methanol to methyl propionate conversion was 90%. The selectivity of the ethene to methyl propionate conversion was found to be almost 100%, while the average conversion rate was 900 mol/g.at.Pd hour.

Example 4

In a virtually analogous manner to that described in Example 1, an experiment was carried out in which 2 mmol triphenyl phosphine sulphide was used. The reaction time was 1.5 hours. The methanol to methyl propionate conversion was 93%. At the end of the reaction, the initially added acid was found (by the aforesaid method) to be still present in almost its entirety in the reaction mixture. The selectivity of the ethene to methyl propionate conversion was found to be almost 100%, while the average conversion rate was 1550 mol/g.at.Pd hour.

An experiment was carried out in the same way, but with a reaction time of 0.5 hours. The initially added acid was found (by the aforesaid method) to be still present in almost its entirety in the reaction mixture. The methanol to methyl propionate conversion was 40%. The selectivity of the ethene to methyl propionate conversion was found to be almost 100%, while an average conversion rate of 2000 mol/g.at.Pd hour was found.

Example 5

In a virtually analogous manner to that described in Example 1, an experiment was carried out in which 0.5 mmol triphenyl phosphine sulphide was used. The reaction time was 1.5 hours. The methanol to methyl propionate conversion was 98%. The initially added acid was found (by the aforesaid method) to be still present in almost ( 95%) its entirety in the reaction mixture. The selectivity of the ethene to methyl propionate conversion was found to be almost 100%, while the average conversion rate was 1640 mol/g.at.Pd hour.

Example 6

In a virtually analogous manner to that described in Example 1, an experiment was carried out in which 5 mmol triphenyl phosphine sulphide was used. The reaction time was 2.5 hours. The methanol to methyl propionate conversion was 92%. The initially added acid was found (by the aforesaid method) to be still present in its entirety in the reaction mixture. The selectivity of the ethene to methyl propionate conversion was found to be almost 100% while the average conversion rate was 920 mol/g.at.Pd hour.

Example 7

In a virtually analogous manner to that described in Example 1, an experiment was carried out in which 10 mmol N-methyl pyrrolidone was used. The reaction time was 2.5 hours. The methanol to methyl propionate conversion was 93%. The initially added acid was found (by the aforesaid method) to be still present in its entirety in the reaction mixture. The selectivity of the ethene to methyl propionate conversion was found to be almost 100% while the average conversion rate was 930 mol/g.at.Pd hour.

Example 8

In a virtually analogous manner to that described in Example 1, an experiment was carried out in which 5 mmol diphenyl acetamide was used. The reaction time was 2.5 hours. The methanol to methyl propionate conversion was 99%. The initially added acid was found (by the aforesaid method) to be still present almost ( 85%) in its entirety in the reaction mixture. The selectivity of the ethene to methyl propionate conversion was found to be almost 100% while the average conversion rate was 1000 mol/g.at.Pd hour.

A comparative experiment carried out in the same way under identical conditions and with the same reaction time of 2.5 hours, but without a stabilizer. The methanol to methyl propionate conversion was now 95%. At the end of the reaction only 35% of the initially added acid was found by the aforesaid method. The selectivity of the ethene to methyl propionate conversion was found to be almost 100%, while the average conversion rate was 900 mol/g.at.Pd hour.

A second comparative experiment was carried out in the same way under identical conditions, but without a stabilizer and with a reaction time of 0.5 hours. The methanol to methyl propionate conversion was now 50%, while only 50% of the initially added acid was found by the aforesaid method in the reaction mixture. The selectivity of the ethene to methyl propionate conversion was almost 100%, while the average conversion rate was 2500 mol/g.at.Pd hour.

From the results of the described experiments in Examples 4 and 8, it is clear that the average conversion rate, drops relatively much more sharply in the absence of a stabilizer according to the present invention than in the presence of such a stabilizer.

## Claims

**1.** A process for the carbonylation of olefinically unsaturated compounds with carbon monoxide in the presence of a hydroxyl-group-containing compound such as water, an alcohol or a carboxylic acid, a palladium catalyst, an organic phosphine according to the formula

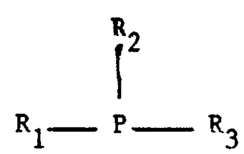

I

wherein $R_1$, $R_2$ and $R_3$ each represent an optionally substituted aryl group, and an acid with a pKa value 2, with the exception of hydrohalogenic acids and carboxylic acids, as promoter, characterized in that a catalyst stabilizer is used.

**2.** A process according to claim 1, characterized in that compounds according to the general formula

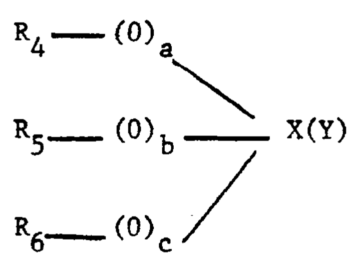

II

wherein X is selected from P, As or Sb, wherein Y is selected from O, S or Se, wherein a, b and c each are 0 or 1, $R_4$, $R_5$ and $R_6$ are the same or different and represent optionally substituted hydrocarbyl groups, or wherein either a and b = 0 and c = 0 or 1 and $R_4$ and $R_5$ together with X form a heterocyclic group, or a, b and c = 0 and $R_4$, $R_5$ and $R_6$ together with X form an optionally substituted heterocyclic group, and/or to the general formula

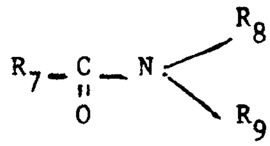

III

wherein $R_7$ represents an optionally substituted hydrocarbyl group and wherein $R_8$ and $R_9$ are each the same or different and have the aforesaid meaning of $R_7$ or together with N form an optionally substituted heterocyclic ring, or wherein $R_7$ and $R_8$ or $R_9$ together with the moiety

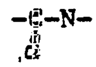

form a heterocyclic ring such as a pyrrolidon or caprolactam ring and/or compounds comprising two or more moieties as represented by formulae II and III are used as catalyst stabilizers.

3. A process according to claim 2, characterized in that the optionally substituted hydrocarbyl groups in the definitions of $R_4$ to $R_9$ are alkyl, cycloalkyl, aryl, aralkyl or alkaryl groups comprising up to 30 carbon atoms.

4. A process according to claim 2 or 3, characterized in that the heterocyclic group comprises up to 21 carbon atoms.

5. A process according to any one of claims 1-4, characterized in that stabilizers are used according to formula II, wherein a, b and c = 0, wherein X is phosphorus, wherein Y is oxygen or sulphur and wherein $R_4$, $R_5$ and $R_6$ are alkyl groups comprising 1-4 carbon atoms, or cycloalkyl, aryl or aralkyl groups comprising 5-12 carbon atoms and preferably represent alkyl, phenyl, cyclohexyl or benzyl groups.

6. A process according to claim 5, characterized in that Y represents oxygen, while $R_4$, $R_5$ and $R_6$ represent alkyl groups, or Y represents sulphur, while $R_4$, $R_5$ and $R_6$ represent optionally substituted phenyl groups.

7. A process according to claim 5 or 6, characterized in that the hydrocarbyl group is substituted by one or more chlorine, alkoxy groups, carboxylic acid (ester) groups or sulphonic or sulphoxide groups.

8. A process according to any one of claims 5-7, characterized in that use is made of one or more of trimethyl phosphine oxide, triethyl phosphine oxide, tri-n-propyl phosphine oxide, tri-n-butyl phosphine oxide, triphenyl phosphine oxide, tri-p-tolyl phosphine oxide, tri-p-tolyl phosphine sulphide, tricyclohexyl phosphine oxide, tricyclohexyl phosphine sulphide, diphenyl ethyl phosphine oxide, tri(1-naphthyl) phosphine oxide, tri(1-naphthyl) phosphine sulphide, 1-phenyl phosphalane sulphide, 1-phenyl phosphorinane sulphide, trimethyl phosphine sulphide, tri(4-chlorophenyl) phosphine sulphide, triphenyl phosphine sulphide, tri-n-butyl phosphine sulphide, triphenyl arsine sulphide, triphenyl phosphine selenide or tri(1-naphthyl) phosphine selenide as stabilizer.

9. A process according to any one of claims 2-4, characterized in that stabilizers according to formula III are used, wherein $R_7$, $R_8$ and $R_9$ represent an alkyl group of 1-4 carbon atoms, a cylcoalkyl, aryl or aralkyl group comprising 5-12 carbon atoms, and preferably an alkyl, phenyl, benzyl or cyclohexyl group, or wherein $R_7$, $R_8$ or $R_9$ together with the moiety -C-N- form a heterocyclic ring.

10. A process according to claim 9, characterized in that

N,N'-dimethyl acetamide, N,N'-diethyl acetamide, N,N'-diethyl propionamide, N,N'-diethyl butamide, N,N'-dipropyl propionamide, N,N'-di(isopropyl)-propionamide, N,N'-di(isopropyl) acetamide, N,N'-diphenyl acetamide, N-methyl pyrrolidone and N-methyl caprolactam are used as stabilizers.

**11.** A process according to any one of claims 1-10, characterized in that a catalyst stabilizer is used in a quantity of 0.01 to 200 mol per gram-atom palladium.

**12.** A process according to any one of claims 1-11, characterized in that acids which comprise a non-coordinating anion are used as promoters.

**13.** A process according to claim 12, characterized in that acids are used which comprise $PF_6^-$, $SbF_6^-$, $BF_4^-$ or $ClO_4^-$ as anions.

**14.** A process according to claim 12 or 13, characterized in that acids are used according to the general formula

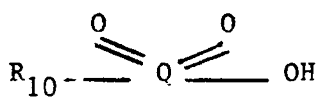

IV

wherein Q represents sulphur or chlorine and wherein, if Q is chlorine, $R_{10}$ represents oxygen, and if Q is sulphur, $R_{10}$ represents an OH-group or an optionally substituted hydrocarbyl group.

**15.** A process according to any one of claims 1-14, characterized in that palladium acetate is used.

**16.** A process according to claims 1-15, characterized in that a quantity of palladium compound of $10^{-5}$ to $10^{-1}$ gram-atom palladium per mol olefinically unsaturated compound is used.

**17.** Catalyst systems for the selective carbonylation of olefinically unsaturated compounds in the presence of a hydroxyl-group-containing compound comprising
(a) a palladium catalyst,
(b) an organic phosphine,
(c) an acid with a pKa value < 2, with the exception of hydrohalogenic acids and carboxylic acids, as promoter, and
(d) a catalyst stabilizer.

**18.** Catalyst systems according to claim 17, characterized in that one or more compounds according to the general formulae

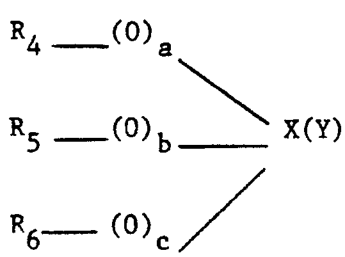

II

wherein X is selected from P, As or Sb, wherein Y is selected from O, S or Se, wherein a, b and c each are 0 or 1, $R_4$, $R_5$ and $R_6$ are the same or different and represent optionally substituted hydrocarbyl groups, or wherein either a and b = 0 and c = 0 or 1 and $R_4$ and $R_5$ together with X form a

heterocyclic group, or a, b and c = 0 and $R_4$, $R_5$ and $R_6$ together with X form an optionally substituted heterocyclic group, and/or to the general formula

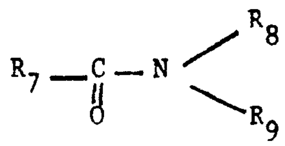

III

wherein $R_7$ represents an optionally substituted hydrocarbyl group and wherein $R_8$ and $R_9$ are each the same or different and have the aforesaid meaning of $R_7$ or together with N form an optionally substituted heterocyclic ring, or wherein $R_7$ and $R_8$ or $R_9$ together with the moiety

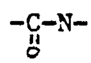

form a heterocyclic ring such as a pyrrolidon or caprolactam ring and/or compounds comprising two or more moieties as represented by formulae II and III are used as catalyst stabilizers.

**19.** Catalyst systems according to claim 17 or 18, characterized in that use is made of one or more of trimethyl phosphine oxide, triethyl phosphine oxide, tri-n-propyl phosphine oxide, tri-n-butyl phosphine oxide, triphenyl phosphine oxide, tri-p-tolyl phosphine oxide, tri-p-tolyl phosphine sulphide, tricyclohexyl phosphine oxide, tricyclohexyl phosphine sulphide, diphenyl ethyl phosphine oxide, tri(1-naphthyl) phosphine oxide, tri(1-naphthyl) phosphine sulphide, 1-phenyl

**20.** Catalyst systems according to any one of claims 17-19, characterized in that stabilizers according to formula III are used, wherein $R_7$, $R_8$ and $R_9$ represent an alkyl group of 1-4 carbon atoms, a cycloalkyl, aryl or aralkyl group comprising 5-12 carbon atoms, and preferably an alkyl, phenyl, benzyl or cyclohexyl group, or wherein $R_7$, $R_8$ or $R_9$ together with the moiety

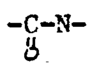

form a heterocyclic ring.

**21.** Catalyst systems according to claim 20, characterized in that
N,N'-dimethyl acetamide, N,N'-diethyl acetamide,
N,N'-diethyl propionamide, N,N'-diethyl butamide,
N,N'-dipropyl propionamide, N,N'-di(isopropyl)-propionamide, N,N'-di(isopropyl) acetamide,
N,N'-diphenyl acetamide, N-methyl pyrrolidone and/or
N-methyl caprolactam are used as stabilizer.

**22.** Catalyst systems according to any one of claims 17-21, characterized in that they contain stabilizer in a quantity of 0.01 to 200 mol per gram-atom palladium.

**23.** Catalyst systems according to any one of claims 17-22, characterized in that they comprise acids which contain a non-coordinating anion as promoter.

**24.** Catalyst systems according to claim 23, characterized in that the acids comprise $PF_6^-$, $SbF_6^-$, $BF_4^-$ or $ClO_4^-$ as anions.

**25.** Catalyst systems according to claim 23, characterized in that they comprise acids according to the general formula

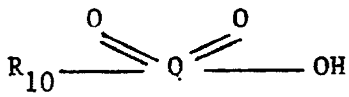

IV

wherein Q represents sulphur or chlorine and wherein, if Q is chlorine, $R_{10}$ represents oxygen and if Q is sulphur, $R_{10}$ represents an OH-group or an optionally substituted hydrocarbyl group.

## Revendications

1. Un procédé pour la carbonylation de composés oléfiniquement insaturés avec de l'oxyde de carbone en présence d'un composé contenant un groupe hydroxyle tel que l'eau, un alcool ou un acide carboxylique, d'un catalyseur au palladium, d'une phosphine organique de la formule

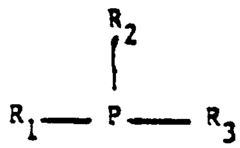

I

où $R_1$, $R_2$ et $R_3$ représentent chacun un groupe aryle éventuellement substitué, et d'un acide ayant un pKa au-dessous de 2, à l'exception des acides halogénhydriques et des acides carboxyliques, comme promoteur, caractérisé en ce qu'on utilise un stabilisant du catalyseur.

2. Un procédé selon la revendication 1, caractérisé en ce que, comme stabilisants du catalyseur, on utilise des composés de la formule générale

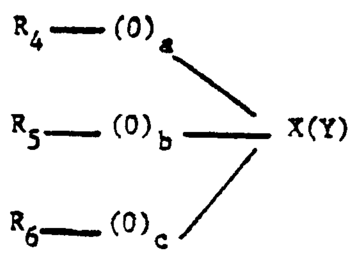

II

où X est choisi parmi P, As ou Sb, Y est choisi parmi O, S ou Se, a, b et c sont chacun 0 ou 1, $R_4$, $R_5$ et $R_6$ sont identiques ou différents et représentent des groupes hydrocarbyle éventuellement substitués, ou a et b = 0 et c = 0 ou 1 et $R_4$ et $R_5$ en même temps que X forment un groupe hétérocyclique, ou a, b et c = 0 et $R_4$, $R_5$ et $R_6$ en même temps que X forment un groupe hétérocycliqueéventuellement substitué, et/ou de la formule générale

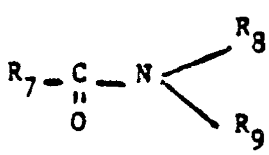

III

où $R_7$ représente un groupe hydrocarbyle éventuellement substitué et $R_8$ et $R_9$ sont identiques ou différents et ont chacun la signification indiquée ci-dessus pour $R_7$ ou en même temps que N forment un noyau hétérocyclique éventuellement substitué, ou $R_7$ et $R_8$ ou $R_9$ en même temps que la portion

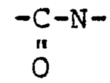

forment un noyau hétérocyclique tel qu'un noyau de pyrrolidone ou de caprolactame et/ou des composés comprenant deux portions ou plus telles que représentées par les formules II et III.

3. Un procédé selon la revendication 2, caractérisé en ce que les groupes hydrocarbyle éventuellement substitués dans les définitions de $R_4$ à $R_9$ sont des groupes alcoyle, oycloalcoyle, aryle, aralcoyle ou alcaryle comprenant jusqu'à 30 atomes de carbone.

4. Un procédé selon la revendication 2 ou 3, caractérisé en ce que le groupe hétérocyclique comprend jusqu'à 21 atomes de carbone.

5. Un procédé selon l'une quelconque des revendications 1-4, caractérisé en ce qu'on utilise des stabilisants de formule II, où a, b et c = 0, x est du phosphore, Y est de l'oxygène ou du soufre et $R_4$, $R_5$ et $R_6$ sont des groupes alcoyle comprenant 1-4 atomes de carbone ou des groupes cycloalcoyle, aryle ou aralcoyle comprenant 5-12 atomes de carbone et de préférence représentent des groupes alcoyle, phényle, cyclohexyle ou benzyle.

6. Un procédé selon la revendication 5, caractérisé en ce que Y représente de l'oxygène, tandis que $R_4$, $R_5$ et $R_6$ représentent des groupes alcoyle, ou Y représente du soufre, tandis que $R_4$, $R_5$ et $R_6$ représentent des groupes phényle éventuellement substitués.

7. Un procédé selon la revendication 5 ou 6, caractérisé en ce que le groupe hydrocarbyle est substitué par un ou plusieurs atomes de chlore, groupes alcoxy, groupes acide (ester) carboxylique ou groupes sulfoniques ou sulfoxyde.

8. Un procédé selon l'une quelconque des revendications 5-7, caractérisé en ce qu'on utilise comme stabilisant un ou plusieurs des composés suivants :
oxyde de triméthyl phosphine, oxyde de triéthyl phosphine, oxyde de tri-n-propyl phosphine, oxyde de tri-n-butyl phosphine, oxyde de triphényl phosphine, oxyde de tri-p-tolyl phosphine, sulfure de tri-p-tolyl phosphine, oxyde de tricyclohexyl phosphine, sulfure de tricyclohexyl phosphine, oxyde de diphényl éthyl phosphine, oxyde de tri(1-naphtyl) phosphine, sulfure de tri(1-naphtyl) phosphine, sulfure de 1-phényl phosphalane, sulfure de 1-phényl phosphorinane, sulfure de triméthyl phosphine, sulfure de tri-(4-chlorophényl) phosphine, sulfure de triphényl phosphine, sulfure de tri-n-butyl phosphine, sulfure de triphényl arsine, séléniure de triphényl phosphine ou séléniure de tri(1-naphtyl) phosphine.

9. Un procédé selon l'une quelconque des revendications 2-4, caractérisé en ce qu'on utilise des stabilisants de formule III, où $R_7$, $R_8$ et $R_9$ représentent un groupe alcoyle de 1-4 atomes de carbone, un groupe cycloalcoyle, aryle ou aralcoyle comprenant 5-12 atomes de carbone, et de préférence un groupe alcoyle, phényle, benzyle ou cyclohexyle, ou $R_7$, $R_8$ ou $R_9$ en même temps que la portion

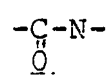

forment un noyau hétérocyclique.

10. Un procédé selon la revendication 9, caractérisé en ce que, comme stabilisants, on utilise les composés suivants : N,N'-diméthyl acétamide, N,N'-diéthyl acétamide, N,N'-diéthyl propionamide, N,N'-diéthyl butamide, N,N'-dipropyl propionamide, N,N'-di(isopropyl) propionamide, N,N'-di(isopropyl) acétamide, N,N'-diphényl acétamide, N-méthyl pyrrolidone et N-méthyl caprolactame.

11. Un procédé selon l'une quelconque des revendications 1-10, caractérisé en ce qu'un stabilisant du catalyseur est utilisé à raison de 0,01 à 200 moles par atome-gramme de palladium.

12. Un procédé selon l'une quelconque des revendications 1-11, caractérisé en ce que des acides qui comprennent un anion non-coordinateur sont utilisés comme promoteurs.

13. Un procédé selon la revendication 12, caractérisé en ce qu'on utilise des acides qui comprennent $PF_6^-$, $SbF_6^-$, $BF_4^-$ ou $ClO_4^-$ comme anions.

14. Un procédé selon la revendication 12 ou 13, caractérisé en ce qu'on utilise des acides de la formule générale

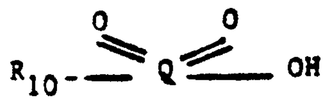

IV

où Q représente du soufre ou du chlore et, si Q est du chlore, alors $R_{10}$ représente de l'oxygène, et si Q est du soufre, alors $R_{10}$ représente un groupe OH ou un groupe hydrocarbyle éventuellement substitué.

15. Un procédé selon l'une quelconque des revendications 1-14, caractérisé en ce qu'on utilise de l'acétate de palladium.

16. Un procédé selon l'une quelconque des revendications 1-15, caractérisé en ce qu'on utilise une quantité de composé du palladium de $10^{-5}$ à $10^{-5}$ atomegramme de palladium par mole de composé oléfiniquement insaturé.

17. Des systèmes catalytiques pour la carbonylation sélective de composés oléfiniquement insaturés en présence d'un composé contenant un groupe hydroxyle, comprenant
   (a) un catalyseur au palladium,
   (b) une phosphine organique,
   (c) un acide ayant un pka au-dessous de 2, à l'exception des acides halogénhydriques et des acides carboxyliques, comme promoteur, et
   (d) un stabilisant du catalyseur.

18. Des systèmes catalytiques selon la revendication 17, caractérisés en ce qu'on utilise comme stabilisants du catalyseur un ou plusieurs composés de la formule générale

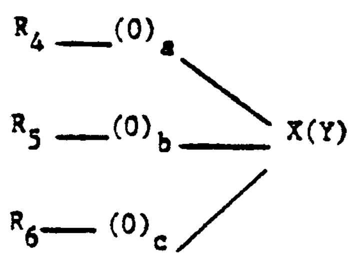

II

où X est choisi parmi P, As ou Sb, Y est choisi parmi O, S ou Se, a, b et c sont chacun 0 ou 1, $R_4$, $R_5$ et $R_6$ sont identiques ou différents et représentent des groupes hydrocarbyle éventuellement substitués, ou a et b = 0 et c = 0 ou 1 et $R_4$ et $R_5$ en même temps que X forment un groupe hétérocyclique, ou a, b et c = 0 et $R_4$, $R_5$ et $R_6$ en même temps que X forment un groupe hétérocyclique éventuellement substitué, et/ou de la formule générale

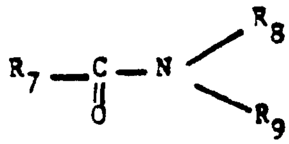

III

où $R_7$ représente un groupe hydrocarbyle éventuellement substitué et $R_8$ et $R_9$ sont identiques ou différents et ont chacun la signification indiquée pour $R_7$ ou en même temps que N forment un noyau hétérocyclique éventuellement substitué, ou R7 et $R_8$ ou $R_9$ en même temps que la portion

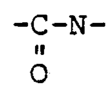

forment un noyau hétérocyclique tel qu'un noyau de pyrrolidone ou de caprolactame et/ou des composés comprenant deux portions ou plus telles que représentées par les formules II et III.

**19.** Des systèmes catalytiques selon la revendication 17 ou 18, caractérisés en ce qu'on utilise un ou plusieurs des composés suivants : oxyde de triméthyl phosphine, oxyde de triéthyl phosphine, oxyde de tri-n-propyl phosphine, oxyde de tri-n-butyl phosphine, oxyde de triphényl phosphine, oxyde de tri-p-tolyl phosphine, sulfure de tri-p-tolyl phosphine, oxyde de tricyclohexyl phosphine, sulfure de tricyclohexyl phosphine, oxyde de diphényl éthyl phosphine, oxyde de tri(1-naphtyl) phosphine, sulfure de tri(1-naphtyl) phosphine, sulfure de 1-phényl phosphalane, sulfure de 1-phényl phosphorinane, sulfure de triméthyl phosphine, sulfure de tri(4-chlorophényl) phosphine, sulfure de triphényl phosphine, sulfure de tri-n-butyl phosphine, sulfure de triphényl arsine, séléniure de triphényl phosphine ou séléniure de tri(1-naphtyl) phosphine.

**20.** Des systèmes catalytiques selon l'une quelconque des revendications 17-19, caractérisés en ce qu'on utilise des stabilisants de formule III, où $R_7$, $R_8$ et $R_9$ représentent un groupe alcoyle de 1-4 atomes de carbone, un groupe cycloalcoyle, aryle ou aralcoyle comprenant 5-12 atomes de carbone, et de préférence un groupe alcoyle, phényle, benzyle ou cyclohexyle, ou $R_7$, $R_8$ ou $R_9$ en même temps que la portion

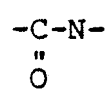

forment un noyau hétérocyclique.

**21.** Des systèmes catalytiques selon la revendication 20, caractérisés en ce qu'on utilise comme stabilisant le N,N'-diméthyl acétamide, le N,N'-diéthyl acétamide, le N,N'-diéthyl propionamide, le N,N'-diéthyl butamide, le N,N'-dipropyl propionamide, le N,N'-di(isopropyl) propionamide, le N,N'-di(isopropyl) acétamide, le N,N'-diphényl acétamide, la N-méthyl pyrrolidone et/ou le N-méthyl caprolactame.

**22.** Des systèmes catalytiques selon l'une quelconque des revendications 17-21, caractérisés en ce qu'il contiennent le stabilisant à raison de 0,01 à 200 moles par atome-gramme de palladium.

**23.** Des systèmes catalytiques selon l'une quelconque des revendications 17-22, caractérisés en ce qu'ils comprennent des acides qui contiennent un anion non-coordinateur comme promoteur.

**24.** Des systèmes catalytiques selon la revendication 23, caractérisés en ce que les acides comprennent $PF_6^-$, $SbF_6^-$, $BF_4^-$ ou $ClO_4^-$ comme anions.

**25.** Des systèmes catalytiques selon la revendication 23, caractérisés en ce qu'ils comprennent des acides de la formule générale

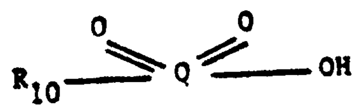

IV

où Q représente du soufre ou du chlore et; si Q est du chlore, alors $R_{10}$ représente de l'oxygène et, si Q est du soufre, alors $R_{10}$ représente un groupe OH ou un groupe hydrocarbyle éventuellement substitué.

## Patentansprüche

1. Verfahren zur Carbonylierung von olefinisch ungesättigten Verbindungen mit Kohlenmonoxid in Gegenwart einer Hydroxylgruppe(n) enthaltenen Verbindung wie Wasser, ein Alkohol oder eine Carbonsäure, eines Palladiumkatalysators, eines organischen Phosphins entsprechend der Formel

   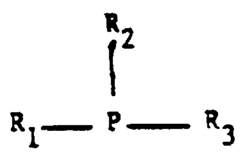

   I

   in der $R_1$, $R_2$ und $R_3$ jeweils eine gegebenenfalls substituierte Arylgruppe bedeuten, und einer Säure mit einem pKa-Wert < 2, ausgenommen Halogenwasserstoffsäuren und Carbonsäuren, als Promotor, dadurch gekennzeichnet, daß ein Stabilisator für den Katalysator verwendet wird.

2. Verfahren nach Anspruch 1, dadurch gekennzeichnet, daß Verbindungen der allgemeinen Formel

   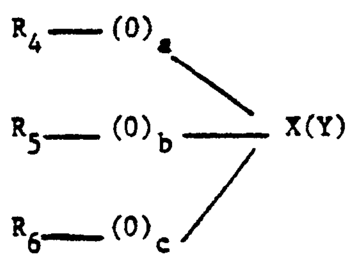

   II

   worin X aus P, As oder Sb ausgewählt ist, worin Y aus O, S oder Se ausgewählt ist, worin a, b und c jeweils 0 oder 1 bedeuten, $R_4$, $R_5$ und $R_6$ gleich oder verschieden sind und gegebenenfalls substituierte Kohlenwasserstoffgruppen bedeuten oder worin entweder a und b = 0 und c = 0 oder 1 und $R_4$ und $R_5$ zusammen mit X eine heterocyclische Gruppe bilden oder a, b und c = 0 und $R_4$, $R_5$ und $R_6$ zusammen mit X eine gegebenenfalls substituierte heterocyclische Gruppe bilden

   und/oder der allgemeinen Formel

   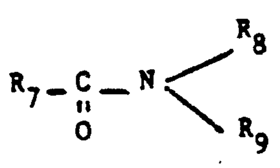

   III

   worin $R_7$ eine gegebenenfalls substituierte Kohlenwasserstoffgruppe bedeutet und worin $R_8$ und $R_9$ jeweils gleich oder verschieden sind und die vorgenannte Bedeutung von $R_7$ haben oder zusammen mit N einen gegebenenfalls substituierten heterocyclischen Ring bilden oder worin $R_7$ und $R_8$ oder $R_9$

zusammen mit der Gruppierung

$$-\underset{\underset{O}{\|}}{C}-N-$$

einen heterocyclischen Ring wie einen Pyrrolidon- oder Caprolactamring bilden und/oder Verbindungen, die zwei oder mehr Molekülteile, wie durch die Formeln II und III wiedergegeben, enthalten, als Stabilisatoren für den Katalysator verwendet werden.

**3.** Verfahren nach Anspruch 2, dadurch gekennzeichnet, daß die gegebenenfalls substituierten Kohlenwasserstoffgruppen in den Definitionen für $R_4$ bis $R_9$ Alkyl-, Cycloalkyl-, Aryl-, Aralkyl-oder Alkarylgruppen mit bis zu 30 Kohlenstoffatomen sind.

**4.** Verfahren nach Anspruch 2 oder 3, dadurch gekennzeichnet, daß die heterocyclische Gruppe bis zu 21 Kohlenstoffatomen enthält.

**5.** Verfahren nach einem der Ansprüche 1 bis 4, dadurch gekennzeichnet, daß Stabilisatoren entsprechend der Formel II eingesetzt werden, worin a, b und c = 0 und worin X Phosphor ist, worin Y Sauerstoff oder Schwefel ist und worin $R_4$, $R_5$ und $R_6$ für Alkylgruppen mit 1 - 4 Kohlenstoffatomen oder für Cycloalkyl-, Aryl- oder Aralkylgruppen mit 5-12 Kohlenstoffatomen stehen und vorzugsweise Alkyl-, Phenyl-, Cyclohexyl- oder Benzylgruppen bedeuten.

**6.** Verfahren nach Anspruch 5, dadurch gekennzeichnet, daß Y für Sauerstoff steht während $R_4$, $R_5$ und $R_6$ Alkylgruppen bedeuten, oder Y für Schwefel steht während $R_4$, $R_5$ und $R_6$ gegebenenfalls substituierte Phenylgruppen bedeuten.

**7.** Verfahren nach Anspruch 5 oder 6, dadurch gekennzeichnet, daß die Kohlenwasserstoffgruppe durch ein/e oder mehrere Chloratome, Alkoxygruppen, Carbonsäure(ester)gruppen oder Sulfonsäure- oder Sulfoxidgruppen substituiert ist.

**8.** Verfahren nach einem der Ansprüche 5 bis 7, dadurch gekennzeichnet, daß ein oder mehrere der folgenden Verbindungen als Stabilisator eingesetzt werden: Trimethylphosphinoxid, Triethylphosphinoxid, Tri-n-propylphosphinoxid, Tri-n-butylphosphinoxid, Triphenylphosphinoxid, Tri-p-tolylphosphinoxid, Trip-tolylphosphinsulfid, Tricyclohexylphosphinoxid, Tricyclohexylphosphinsulfid, Diphenylethylphosphinoxid, Tri(1-naphthyl)-phosphinoxid, Tri(1-naphthyl)phosphinsulfid, 1-Phenylphosphalansulfid, 1-Phenylphosphorinansulfid, Trimethylphosphinsulfid, Tri(4-chlorphenyl)phosphinsulfid, Triphenylphosphinsulfid, Tri-n-butylphosphinsulfid, Triphenylarsinsulfid, Triphenylphosphinselenid oder Tri(1-naphthyl)-phosphinselenid.

**9.** Verfahren nach einem der Ansprüche 2 bis 4, dadurch gekennzeichnet, daß Stabilisatoren entsprechend der Formel III eingesetzt werden, worin $R_7$, $R_8$ und $R_9$ jeweils eine Alkylgruppe mit 1-4 Kohlenstoffatomen, eine Cycloalkyl-, Aryl- oder Aralkylgruppe mit 5-12 Kohlenstoffatomen und vorzugsweise eine Alkyl-, Phenyl-, Benzyl- oder Cyclohexylgruppe bedeuten oder worin $R_7$, $R_8$ oder $R_9$ zusammen mit der Gruppierung

$$-\underset{\underset{O}{\|}}{C}-N-$$

einen heterocyclischen Ring bilden.

**10.** Verfahren nach Anspruch 9, dadurch gekennzeichnet, daß N,N'-Dimethylacetamid, N,N'-Diethylacetamid, N,N'-Diethylpropionamid, N,N'-Diethylbutamid, N,N'-Dipropylpropionamid, N,N'-Di(isopropyl)-propionamid, N,N'-Di(isopropyl)acetamid, N,N'-Di-phenylacetamid, N-Methylpyrrolidon und N-Methylcaprolactam als Stabilisatoren eingesetzt werden.

**11.** Verfahren nach einem der Ansprüche 1 bis 10, dadurch gekennzeichnet, daß ein Stabilisator für den Katalysator in einer Menge von 0,01 bis 200 Mol/gAtom Palladium eingesetzt wird.

**12.** Verfahren nach einem der Ansprüche 1 bis 11, dadurch gekennzeichnet, daß Säuren, die ein nicht-koordinierendes Anion enthalten, als Promotoren verwendet werden.

**13.** Verfahren nach Anspruch 12, dadurch gekennzeichnet, daß Säuren verwendet werden, die $PF_6^-$, $SbF_6^-$, $BF_4^-$ oder $ClO_4^-$ als Anionen umfassen.

**14.** Verfahren nach Anspruch 12 oder 13, dadurch gekennzeichnet, daß Säuren entsprechend der allgemeinen Formel

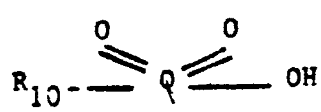

IV

verwendet werden, in der Q Schwefel oder Chlor bedeutet und worin, wenn Q für Chlor steht, $R_{10}$ Sauerstoff bedeutet und wenn Q für Schwefel steht, $R_{10}$ eine OH-Gruppe oder eine gegebenenfalls substituierte Kohlenwasserstoffgruppe bedeutet.

**15.** Verfahren nach einem der Ansprüche 1 bis 14, dadurch gekennzeichnet, daß Palladiumacetat verwendet wird.

**16.** Verfahren nach Anspruch 1 bis 15, dadurch gekennzeichnet, daß eine Menge an Palladiumverbindung von $10^{-5}$ bis $10^{-1}$ gAtom Palladium je Mol olefinisch ungesättigte Verbindung verwendet wird.

**17.** Katalysatorsysteme für die selektive Carbonylierung von olefinisch ungesättigten Verbindungen in Gegenwart einer Hydroxylgruppe(n) enthaltenen Verbindung, umfassend

(a) einen Palladiumkatalysator,
(b) ein organisches Phosphin,
(c) eine Säure mit einem pKa-Wert < 2, ausgenommen Halogenwasserstoffsäuren und Carbonsäuren, als Promotor und
(d) einen Stabilisator für den Katalysator.

**18.** Katalysatorsysteme nach Anspruch 17, dadurch gekennzeichnet, daß eine oder mehrere Verbindungen entsprechend den allgemeinen Formeln

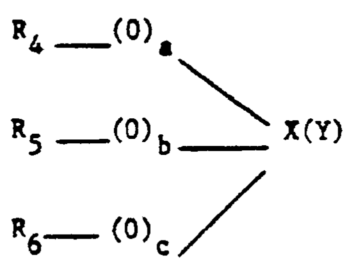

II

worin X aus P, As oder Sb ausgewählt ist, worin Y aus O, S oder Se ausgewählt ist, worin a, b und c jeweils 0 oder 1 bedeuten, $R_4$, $R_5$ und $R_6$ gleich oder verschieden sind und gegebenenfalls substituierte Kohlenwasserstoffgruppen bedeuten oder worin entweder a und b = 0 und c = 0 oder 1 und $R_4$ und $R_5$ zusammen mit X eine heterocyclische Gruppe bilden oder a, b und c = 0 und $R_4$, $R_5$ und $R_6$ zusammen mit X eine gegebenenfalls substituierte heterocyclische Gruppe bilden

und/oder der allgemeinen Formel

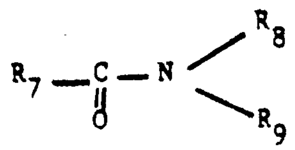

III

worin $R_7$ eine gegebenenfalls substituierte Kohlenwasserstoffgruppe bedeutet und worin $R_8$ und $R_9$ jeweils gleich oder verschieden sind und die vorgenannte Bedeutung von $R_7$ haben oder zusammen mit N einen gegebenenfalls substituierten heterocyclischen Ring bilden oder worin $R_7$ und $R_8$ oder $R_9$ zusammen mit der Gruppierung

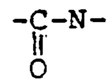

einen heterocyclischen Ring wie einen Pyrrolidon- oder Caprolactamring bilden und/oder Verbindungen, die zwei oder mehr Molekülteile, wie durch die Formeln II und III wiedergegeben, enthalten, als Stabilisatoren für den Katalysator verwendet werden.

**19.** Katalysatorsysteme nach Anspruch 17 oder 18, dadurch gekennzeichnet, daß Gebrauch gemacht wird von einer oder mehreren der folgenden Verbindungen: Trimethylphosphinoxid, Triethylphosphinoxid, Tri-n-propylphosphinoxid, Tri-n-butyl-phosphinoxid, Triphenylphosphinoxid, Tri-p-tolyphosphinoxid, Tri-p-tolylphosphinsulfid, Tricyclohexylphospninoxid, Tricyclohexylphosphinsulfid,Diphenylethylphosphinoxid, Tri(1-naphthyl)phosphinoxid, Tri(1-naphthyl)phosphinsulfid, 1-Phenylphosphalansulfid, 1-Phenylphosphorinansulfid, Trimethylphosphinsulfid, Tri(4-chlorphenyl)-phosphinsulfid, Triphenylphosphinsulfid, Tri-n-butylphosphinsulfid, Triphenylarsinsulfid, Triphenylphosphinselenid oder Tri(1-naphthyl)phosphinselenid.

**20.** Katalysatorsysteme nach einem der Ansprüche 17 bis 19, dadurch gekennzeichnet, daß Stabilisatoren entsprechend der Formel III verwendet werden, worin $R_7$, $R_8$ und $R_9$ jeweils eine Alkylgruppe mit 1-4 Kohlenstoffatomen, eine Cycloalkyl-, Aryloder Aralkylgruppe mit 5-12 Kohlenstoffatomen und vorzugsweise eine Alkyl-, Phenyl-, Benzyl- oder Cyclohexylgruppe bedeuten oder worin $R_7$, $R_8$ oder $R_9$ zusammen mit der Gruppierung

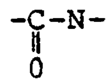

einen heterocyclischen Ring bilden.

**21.** Katalysatorsysteme nach Anspruch 20, dadurch gekennzeichnet, daß N,N'-Dimethylacetamid, N,N'-Diethylacetamid, N,N'-Diethylpropionamid, N,N'-Diethylbutamid, N,N'-Dipropylpropionamid, N,N'-Di(isopropyl)propionamid, N,N'-Di(isopropyl)acetamid, N,N'-Diphenylacetamid, N-Methylpyrrolidon und/oder N-Methylcaprolactam als Stabilisator verwendet werden.

**22.** Katalysatorsysteme nach einem der Ansprüche 17 bis 21, dadurch gekennzeichnet, daß sie den Stabilisator in einer Menge von 0,01 bis 200 Mol/gAtom Palladium enthalten.

**23.** Katalysatorsysteme nach einem der Ansprüche 17 bis 22, dadurch gekennzeichnet, daß sie Säuren, die ein nicht-koordinierendes Anion enthalten, als Promotor umfassen.

**24.** Katalysatorsysteme nach Anspruch 23, dadurch gekennzeichnet, daß die Säuren $PF_6^-$, $SbF_6^-$, $BF_4^-$ oder $ClO_4^-$ als Anionen umfassen.

25. Katalysatorsysteme nach Anspruch 23, dadurch gekennzeichnet, daß sie Säuren der allgemeinen Formel

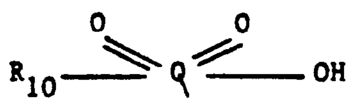

IV

umfassen, worin Q Schwefel oder Chlor bedeutet und worin, wenn Q für Chlor steht, $R_{10}$ Sauerstoff bedeutet und wenn Q für Schwefel steht, $R_{10}$ eine OH-Gruppe oder eine gegebenenfalls substituierte Kohlenwasserstoffgruppe bedeutet.